(19) Europäisches Patentamt / European Patent Office / Office européen des brevets

(11) **EP 3 842 504 B1**

(12) **EUROPEAN PATENT SPECIFICATION**

(45) Date of publication and mention
of the grant of the patent:
**31.12.2025 Bulletin 2026/01**

(21) Application number: **19851681.7**

(22) Date of filing: **21.08.2019**

(51) International Patent Classification (IPC):
*C09J 153/02* (2006.01)     *A61F 13/84* (2006.01)
*A61L 15/22* (2006.01)     *A61L 15/58* (2006.01)
*C09J 11/08* (2006.01)

(52) Cooperative Patent Classification (CPC):
(C-Sets available)
**A61L 15/58; C09J 153/02**                    (Cont.)

(86) International application number:
**PCT/JP2019/032576**

(87) International publication number:
**WO 2020/040175 (27.02.2020 Gazette 2020/09)**

(54) **HOT-MELT ELASTIC ADHESIVE**

ELASTISCHER HEISSSCHMELZKLEBSTOFF

ADHÉSIF ÉLASTIQUE THERMOFUSIBLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.08.2018   JP 2018156292**

(43) Date of publication of application:
**30.06.2021   Bulletin 2021/26**

(73) Proprietor: **Zeon Corporation
Tokyo 100-8246 (JP)**

(72) Inventors:
• **ISHII, Yuta
Tokyo 100-8246 (JP)**

• **HASHIMOTO, Sadaharu
Tokyo 100-8246 (JP)**

(74) Representative: **Kraus & Lederer PartGmbB
Thomas-Wimmer-Ring 15
80539 München (DE)**

(56) References cited:
WO-A1-2014/050664     WO-A2-02/18512
JP-A- 2010 254 983     JP-B2- 5 402 780
US-A- 6 103 814     US-A- 6 140 433
US-A1- 2004 005 834     US-A1- 2017 002 190
US-A1- 2018 208 809     US-B2- 8 163 824

**(Cont. next page)**

EP 3 842 504 B1

(52) Cooperative Patent Classification (CPC): (Cont.)

C-Sets
**A61L 15/58, C08L 35/06**

## Description

TECHNICAL FIELD

The present invention relates to a hot-melt adhesive and, more particularly, to a hot-melt adhesive excellent in creep resistance and adhesion.

BACKGROUND ART

A hot-melt adhesive is adhesive which can efficiently bond a variety of products because of their short solidification time, and has high safety to human bodies because it is solvent-free. For example, when producing an adhesive for sealing for a paper, a cardboard, a film for a food, a clothing, an electronic device, a cosmetic and the like, or producing a sanitary product such as a paper diaper and a sanitary product, a hot-melt adhesive is used as an adhesive for bonding a member for constituting them, or as an adhesive constituting an adhesive layer of an adhesive tape or a label.

As a hot-melt adhesive, for example, Patent Document 1 proposes a hot-melt adhesive composition comprising a block copolymer composition containing a block copolymer A represented by the following general formula (A) and a block copolymer B represented by the following general formula (B), and a tackifying resin, wherein a weight ratio (A/B) of the block copolymer A and the block copolymer B in the block copolymer composition is 10/90 to 90/10, and an aromatic monomer unit content in the tackifying resin is 1 to 70% by weight.

$$Ar1^a\text{-}D^a\text{-}Ar2^a \qquad (A)$$

$$(Ar^b\text{-}D^b)_n\text{-}X \qquad (B)$$

In the general formulas (A) and (B), $Ar1^a$ and $Ar^b$ are each an aromatic vinyl polymer block having a weight average molecular weight of 6000 to 20000, $Ar2^a$ is an aromatic vinyl polymer block having a weight average molecular weight of 22000 to 400000, and $D^a$ and $D^b$ are each a conjugated diene polymer block having a vinyl bond content of 1 to 20 mol%, X is a single bond or a residue of a coupling agent, and "n" is an integer of 2 or more.

JP 2010 254983 A relates to a hot-melt adhesive composition, and more specifically, for bonding members constituting paper diapers, sanitary products, and the like, and suitably used as a pressure-sensitive adhesive for pressure-sensitive adhesive labels, etc., at a relatively low temperature.

US 2004/005834 Al is directed to elastomeric adhesive compositions which significantly improve decay and adhesion properties of elastomeric laminates.

RELATED ART

PATENT DOCUMENTS

Patent Document 1: JP-A-2010-254983

SUMMARY OF THE INVENTION

PROBLEM TO BE SOLVED BY THE INVENTION

However, in the hot-melt adhesive used in manufacturing hygiene products such as paper diapers and sanitary products, not only an excellent adhesion is required, but also an excellent elasticity has been required as compared with a conventional one.

In view of such circumstances, it is an object according to the present invention to provide a hot-melt adhesive which is excellent in creep resistance and adhesion.

MEANS FOR SOLVING THE PROBLEM

As a result of extensive studies to achieve the above object, the present inventors have found that, by using a block copolymer in which a melt flow rate, a content of an aromatic vinyl monomer unit, and a Shore A hardness are appropriately adjusted, the obtained hot-melt adhesive is excellent in creep resistance and is also excellent in adhesion, thereby completing the present invention.

That is, according to the present invention, there is provided a ho t-melt adhesive comprising a block copolymer_composition, wherein

the block copolymer composition has a melt flow rate measure d in accordance with ISO 1133 [G condition, 200°C, 5 kg] of 40 to 200 g/10 min,

the block copolymer composition consists of two or more bloc k copolymers,

each of the block copolymers contain an aromatic vinyl poly mer block and a conjugated diene polymer block,

the block copolymer composition comprises, as the block copo lymer, a block copolymer A represented by the following general f ormula (A) and at least one selected from the group consisting of a block copolymer B represented by the following general formula (B) and a block copolymer C represented by the following general formula (C),

General formula (A): $Ar1^a\text{-}D^a\text{-}Ar2^a$

in the general formula (A), $Ar1^a$ is an aromatic vinyl polymer block having a weight average molecular weight of 5000 to 20000, $Ar2^a$ is an aromatic vinyl polymer block having a weight average m olecular weight of 22000 to 400000, and $D^a$ is a conjugated diene p olymer block,

General formula (B): $(Ar^b\text{-}D^b)_n\text{-}X$

in the general formula (B), $Ar^b$ is an aromatic vinyl polymer block having a weight average molecular weight of 5000 to 20000, $D^b$ is a conjugated diene polymer block, X is a single bond, n is 2, and the sum of the weight average molecular weights of the two conjugated diene polymer blocks $D^b$ is equal to the weight average molecular weight of the conjugated diene polymer block $D^a$ of the b lock copolymer A,

General formula (C): $Ar^c\text{-}D^c$

in the general formula (C), $Ar^c$ represents an aromatic vinyl polymer block and $D^c$ represents a conjugated diene polymer block, and the weight average molecular weight of the conjugated diene polymer block $D^c$ is equal to the weight average molecular weight o f the conjugated diene polymer block $D^a$ of the block copolymer A,

each of a weight average molecular weight of a polymer block and a weight average molecular weight of an entire block polymer is determined as a value in terms of polystyrene by measurement o f high performance liquid chromatography,

a content of an aromatic vinyl monomer unit in the block copo lymer composition is 30 to 80% by weight with respect to the total monomer unit constituting the block copolymer composition, and

the block copolymer composition has a Shore A hardness determined in accordance with ISO 7619 of 65 or less

In the hot-melt adhesive according to the present invention, the conjugated diene polymer block preferably contains an isoprene unit.

The hot-melt adhesive according to the present invention preferably comprises, as the block copolymer, the block copolymer A and the block copolymer B.

The hot-melt adhesive according to the present invention preferably further comprises a tackifying resin.

The melt adhesive according to the present invention preferably compris es a non-aromatic tackifying resin having an aromatic monomer uni t content of less than 1% by weight as the tackifying resin.

In another embodiment the present invention is directed to the use of the hot-melt adhesive according to the invention in the ma nufacture of a paper diaper.

The hot-melt adhesive according to the present invention is preferably used for a paper diaper.

EFFECTS OF INVENTION

According to the present invention, it is possible to provide a hot-melt adhesive which is excellent in creep resistance and adhesion.

DESCRIPTION OF EMBODIMENTS

The hot-melt adhesive according to the present invention comprises a block copolymer.

(Block copolymer)

The block copolymer contained in the hot-melt adhesive according to the present invention has a melt flow rate of 40 to 200 g/10 min, preferably 42.5 to 175 g/10 min, and more preferably 45 to 150 g/10 min. When the melt flow rate of the block copolymer is within the above range, the hot-melt adhesive according to the present invention is excellent in creep resistance and adhesion. If the melt flow rate of the block copolymer is too low, a sufficient creep resistance cannot be obtained.

In the present invention, the melt flow rate is determined under a condition of 200°C, 5 kg, according to ISO 1133 (G condition). The melt flow rate of the block copolymer can be controlled by adjusting the weight average molecular weight of the block copolymer and the like.

The Shore A hardness of the block copolymer contained in the hot-melt adhesive according to the present invention is 65 or less, preferably 64 or less, more preferably 63 or less, and may be 40 or more. When the Shore A hardness of the block copolymer is within the above range, the hot-melt adhesive according to the present invention is excellent in creep resistance and adhesion. If the Shore A hardness is too high, a sufficient adhesion cannot be obtained.

The Shore A hardness is determined in accordance with ISO 7619. The Shore A hardness of the block copolymer can be controlled by adjusting the content of an aromatic vinyl monomer unit of the block copolymer, the weight average molecular weight of the aromatic vinyl polymer block in the block copolymer, and the like.

The block copolymer contained in the hot-melt adhesive according to the present invention contains an aromatic vinyl polymer block and a conjugated diene polymer block.

The aromatic vinyl polymer block is a polymer block composed of, as a main repeating unit, an aromatic vinyl monomer unit obtained by polymerizing an aromatic vinyl monomer.

The content of the aromatic vinyl monomer unit in the block copolymer is 30 to 80% by weight, preferably 31 to 70% by weight, and more preferably 32 to 65% by weight, with respect to the total monomer unit constituting the block copolymer. When the content of the aromatic vinyl monomer unit of the block copolymer is within the above range, the hot-melt adhesive according to the present invention is excellent in creep resistance and adhesion. If the content of the aromatic vinyl monomer unit of the block copolymer is too small, a sufficient creep resistance cannot be obtained. The hot-melt adhesive according to the present invention thus contains a block copolymer having a small Shore A hardness despite a relatively large content of aromatic vinyl monomer unit.

The aromatic vinyl monomer used to form the aromatic vinyl monomer unit for the aromatic vinyl polymer block may be any aromatic vinyl compound. Examples of usable aromatic vinyl monomers include styrene, $\alpha$-methylstyrene, 2-methylstyrene, 3-methylstyrene, 4-methylstyrene, 2-ethylstyrene, 3-ethylstyrene, 4-ethylstyrene, 2,4-diisopropylstyrene, 2,4-dimethylstyrene, 4-t-butylstyrene, 5-t-butyl-2-methylstyrene, 2-chlorostyrene, 3-chlorostyrene, 4-chlorostyrene, 4-bromostyrene, 2-methyl-4,6-dichlorostyrene, 2,4-dibromostyrene, vinylnaphthalene, and the like. Among these, use of styrene is preferred. These aromatic vinyl monomers can be used alone or in combination. When a plurality of aromatic vinyl polymer blocks is present, each of them may be composed of the same aromatic vinyl monomer unit or may be composed of different aromatic vinyl monomer units.

The aromatic vinyl block may contain another monomer unit as long as the aromatic vinyl monomer unit is the main repeating unit. Examples of monomers which form monomer unit other than the aromatic vinyl monomer unit contained in the aromatic vinyl block include conjugated diene monomers such as 1,3-butadiene and isoprene (2-methyl-1,3-butadiene), $\alpha,\beta$-unsaturated nitrile monomers, unsaturated carboxylic acid monomers or acid anhydride monomers, unsaturated carboxylic acid ester monomers, non-conjugated diene monomers, and the like. The content of the monomer unit other than the aromatic vinyl monomer unit in the aromatic vinyl block is preferably 20% by weight or less, more preferably 10% by weight or less, particularly preferably substantially 0% by weight. In other words, the aromatic vinyl block is preferably composed of substantially only unit of one or two or more aromatic vinyl monomers, and particularly preferably composed of only styrene unit.

The weight average molecular weight of the aromatic vinyl polymer block is preferably 5000 to 400000, more preferably 6000 to 350000, still more preferably 7000 to 300000, particularly preferably 8000 to 300000, and most preferably 8500 to 200000.

In the present invention, the weight average molecular weight of each polymer block and the weight average molecular weight of the entire block polymer are determined as a value in terms of polystyrene by measurement of high performance liquid chromatography.

Further, the weight average molecular weight of each polymer block and the weight average molecular weight of the entire block polymer can be controlled by adjusting the amount of each monomer to be used for forming each polymer block and the amount of the polymerization initiator to be used when obtaining the block copolymer by the polymerization reaction.

The conjugated diene polymer block is a polymer block composed of, as a main repeating unit, a conjugated diene monomer unit obtained by polymerizing a conjugated diene monomer.

The conjugated diene monomer used to form the conjugated diene monomer unit for the conjugated diene block may be any conjugated diene compound. Examples thereof include 1,3-butadiene, isoprene, 2,3-dimethyl-1,3-butadiene, 2-chloro-1,3-butadiene, 1,3-pentadiene, 1,3-hexadiene, myrcene, farnesene, and the like. Among these, preferred is use of 1,3-butadiene and/or isoprene, and particularly preferred is use of isoprene. These conjugated diene monomers can be used alone or in combination. Furthermore, unsaturated bond of the conjugated diene block may be partially subjected to a hydrogenation reaction.

The conjugated diene block may contain another monomer unit as long as the conjugated diene monomer unit is the main repeating unit. Examples of monomers which form monomer unit other than the conjugated diene monomer unit contained in the conjugated diene block include aromatic vinyl monomers such as styrene and α-methylstyrene, α,β-unsaturated nitrile monomers, unsaturated carboxylic acid monomers or acid anhydride monomers, unsaturated carboxylic acid ester monomers, non-conjugated diene monomers, and the like. The content of the monomer unit other than the conjugated diene monomer unit in the conjugated diene block is preferably 20% by weight or less, more preferably 10% by weight or less, particularly preferably substantially 0% by weight. In other words, the conjugated diene block is preferably composed of substantially only unit of one or two or more conjugated diene monomers, and particularly preferably composed of only isoprene unit.

Although not particularly limited, the vinyl bond content in the conjugated diene block (total proportion of 1,2-vinyl bond unit and 3,4-vinyl bond unit in the total conjugated diene monomer unit included in the conjugated diene block) is preferably 1 to 20 mol%, more preferably 2 to 15 mol%, particularly preferably 3 to 10 mol%. If the vinyl bond content is too large, the flexibility of the block copolymer tends to be impaired.

The weight average molecular weight of the conjugated diene polymer block is preferably 20000 to 400000, more preferably 22500 to 350000, still more preferably 25000 to 300000, and particularly preferably 30000 to 200000.

Although the block copolymer contained in the hot-melt adhesive according to the present invention may contain one or two or more block copolymers, it is preferable that the block copolymer contains a block copolymer which has at least a terminal block and an intermediate block, the terminal block containing a block copolymer composed of an aromatic vinyl polymer block. In other words, it is preferable that the block copolymer contains a block copolymer having an aromatic vinyl polymer block at an end of a polymer chain. When containing the block copolymer in which the terminal block is composed of an aromatic vinyl polymer block as a block copolymer, the hot-melt adhesive is more excellent in creep resistance and adhesion. In the hot-melt adhesive according to the present invention, a part of the terminal blocks of the block copolymer is preferably constituted by an aromatic vinyl polymer block, and it is more preferable that all of the terminal blocks of the block copolymer are constituted by an aromatic vinyl polymer block because of further excellent creep resistance and an adhesion. Note that, in this specification, the terminal block means a block constituting a terminal of a copolymer chain in a block copolymer having at least 3 blocks, and when the copolymer chain is linear, the number of terminal blocks is two. In addition, an intermediate block means a block other than a terminal block in a polymer having at least three blocks.

Although the block copolymer contained in the hot-melt adhesive according to the present invention may contain one or two or more block copolymers, it is preferable that the block copolymer contains a block copolymer which has at least a terminal block and an intermediate block, the intermediate block containing a block copolymer composed of a conjugated diene polymer block. When the block copolymer contains a block copolymer in which an intermediate block is constituted by a conjugated diene polymer block as a block copolymer, the hot-melt adhesive is more excellent in creep resistance and adhesion. In the hot-melt adhesive according to the present invention, a part of the intermediate blocks of the block copolymer is preferably constituted by a conjugated diene polymer block, and it is more preferable that all of the intermediate blocks of the block copolymer are constituted by a conjugated diene polymer block because of further excellent creep resistance and adhesion. In addition, the conjugated diene polymer block preferably contains an isoprene unit, and particularly preferably, the conjugated diene polymer block is composed of only an isoprene unit.

(Block copolymer A)

The hot-melt adhesive according to the present invention contains a block copolymer A in the block copolymer composition because more excellent creep resistance and adhesion can be obtained. The block copolymer A is a copolymer represented by the following general formula (A):

General formula (A) : $Ar1^a\text{-}D^a\text{-}Ar2^a$

(In the formula, $Ar1^a$ is an aromatic vinyl polymer block having a weight average molecular weight of 5000 to 20000, $Ar2^a$ is an aromatic vinyl polymer block having a weight average molecular weight of 22000 to 400000, and $D^a$ is a conjugated diene polymer block.)

The aromatic vinyl polymer block ($Ar1^a$) and the aromatic vinyl polymer block ($Ar2^a$) are polymer blocks composed of aromatic vinyl monomer units obtained by polymerizing an aromatic vinyl monomer as main repeating unit.

The aromatic vinyl monomer used to constitute the aromatic vinyl monomer unit of the aromatic vinyl polymer block ($Ar1^a$) and the aromatic vinyl polymer block ($Ar2^a$) is not particularly limited as long as it is an aromatic vinyl compound, and examples thereof include the same as those of the aromatic vinyl polymer block described above.

The aromatic vinyl polymer block ($Ar1^a$) and the aromatic vinyl polymer block ($Ar2^a$), as long as the aromatic vinyl monomer unit is the main repeating unit, may include another monomer unit, as the monomer constituting the monomer unit other than the aromatic vinyl monomer unit, examples thereof include the same as those of the aromatic vinyl polymer block described above, and the content thereof may be the same as in the aromatic vinyl polymer block described above. Further, it is preferable that the aromatic vinyl polymer block (Ar1ᵃ) and the aromatic vinyl polymer block (Ar2ᵃ) are also substantially composed of only one type or two or more types of aromatic vinyl monomer units, and it is particularly preferable that they are composed of only styrene units.

In the block copolymer A, the aromatic vinyl polymer block (Ar1ᵃ) and the aromatic vinyl polymer block (Ar2ᵃ) differ in weight-average molecular weight. When the hot-melt adhesive according to the present invention contains such an asymmetric block copolymer A, the hot-melt adhesive according to the present invention is further superior in creep resistance and adhesion. In addition, when a conventional hot-melt adhesive contains a block copolymer having a relatively large content of an aromatic vinyl monomer unit, the hardness tends to be high and the adhesion tends to be inferior. When the hot-melt adhesive according to the present invention contains such an asymmetric block copolymer A which has an aromatic vinyl polymer block (Ar2ᵃ) having an extremely limited weight-average molecular weight, the hot-melt adhesive according to the present invention exhibits a more excellent creep resistance and at the same time exhibits a higher adhesion.

The weight average molecular weight of the aromatic vinyl polymer block (Ar1ᵃ) is 5000 to 20000, more preferably 6000 to 18000, and still more preferably 7000 to 16000.

The weight average molecular weight of the aromatic vinyl polymer blocks (Ar2ᵃ) is 22000 to 400000, more preferably 25000 to 300000, and still more preferably 30000 to 200000.

The ratio of the weight average molecular weight between the aromatic vinyl polymer block (Ar1ᵃ) and the aromatic vinyl polymer blocks (Ar2ᵃ) ((Ar1ᵃ):(Ar2ᵃ)) is preferably 1:1 to 1:15, more preferably 1:1.5 to 1:13, and still more preferably 1:4 to 1:10.

The conjugated diene polymer block (Dᵃ) is a polymer block composed of a conjugated diene monomer unit obtained by polymerizing a conjugated diene monomer as a main repeating unit.

The conjugated diene monomer used for constituting the conjugated diene monomer unit of the conjugated diene polymer block (Dᵃ) is not particularly limited as long as it is a conjugated diene compound, and examples thereof include the same as those of the conjugated diene polymer block described above.

The conjugated diene polymer block (Dᵃ), as long as the conjugated diene monomer unit is the main repeating unit, may include another monomer unit, as the monomer constituting the monomer unit other than the conjugated diene monomer unit, examples thereof include the same as those of the conjugated diene polymer block described above, and the content thereof and vinyl bond content may be the same as in the conjugated diene polymer block described above. Further, it is preferable that the conjugated diene polymer block (Dᵃ) is also substantially composed of only one type or two or more types of conjugated diene monomer units, and it is particularly preferable that it is composed of only isoprene unit.

The weight average molecular weight of the conjugated diene polymer block (Dᵃ) is preferably 40000 to 400000, more preferably 45000 to 350000, and still more preferably from 50000 to 300000.

The weight average molecular weight of the block copolymer A (that is, the weight average molecular weight of the entire block copolymer A) is preferably 65000 to 800000, more preferably 70000 to 700000, and still more preferably 75000 to 650000.

The content of the aromatic vinyl monomer unit of the block copolymer A is preferably 30% by weight or more, more preferably 30 to 87% by weight, still more preferably 35 to 85% by weight, and particularly preferably 35 to 80% by weight, with respect the total monomer unit constituting the block copolymer A.

It is preferable that the block copolymer A is obtained by a production method in which a polymer block is formed by sequentially polymerizing an aromatic vinyl monomer, a conjugated diene monomer, and then an aromatic vinyl monomer in this order. Therefore, it is preferable that the block copolymer A does not contain a residue of a coupling agent. Details of such a production method will be described later.

(Block copolymer B)

It is preferable that the hot-melt adhesive according to the present invention further contains a block copolymer B in addition to the block copolymer A because more excellent creep resistance and adhesion can be obtained. The block copolymer B is a copolymer represented by the following general formula (B):

General formula (B) :         $(Ar^b\text{-}D^b)_{n\text{-}X}$

(In the formula, $Ar^b$ is an aromatic vinyl polymer block having a weight average molecular weight of 5000 to 20000, $D^b$ is a conjugated diene polymer block, X is a single bond or a residue of coupling agent, and "n" represents an integer of 2 or more.)

The block copolymer B has a structure in which diblock structures ($Ar^b\text{-}D^b$) of the number of "n" are bonded to each other in a direct single bond or through a residue (X) of a coupling agent. "n" in the general formula (B) represents the number of branches in the block copolymer B. The block copolymer B may be a mixture of two or more block copolymers in which diblock structure is bonded at different numbers. "n" is an integer of two or more, preferably an integer of 2 to 8, more preferably an integer of 2 to 4, and still more preferably 2. The residue (X) of the coupling agent is not particularly limited as long as it is a residue of an n-valent coupling agent (coupling agent in which valent number is "n"), but is preferably a residue of a silicon atom-containing coupling agent, and more preferably a residue of a silane halide or an alkoxysilane. Examples of the coupling agent constituting the residue of the coupling agent include those described later.

The aromatic vinyl polymer block (Ar$^b$) is a polymer block composed of an aromatic vinyl monomer unit obtained by polymerizing an aromatic vinyl monomer as a main repeating unit.

The aromatic vinyl monomer used for constituting the aromatic vinyl monomer unit of the aromatic vinyl polymer block (Ar$^b$) is not particularly limited as long as it is an aromatic vinyl compound, and examples thereof include the same as those of the aromatic vinyl polymer block described above.

The aromatic vinyl polymer block (Ar$^b$), as long as the aromatic vinyl monomer unit is the main repeating unit, may include another monomer unit, as the monomer constituting the monomer unit other than the aromatic vinyl monomer unit, examples thereof include the same as those of the aromatic vinyl polymer block described above, and the content thereof may be the same as in the aromatic vinyl polymer block described above. Further, each aromatic vinyl polymer block in the block copolymer B may be composed of the same aromatic vinyl monomer unit or may be composed of different aromatic vinyl monomer units. In addition, it is preferable that the aromatic vinyl polymer block (Ar$^b$) is also substantially composed of only one type or two or more types of aromatic vinyl monomer units, and it is particularly preferable that it is composed of only styrene unit.

The weight average molecular weight of the aromatic vinyl polymer blocks (Ar$^b$) is 5000 to 20000, more preferably 6000 to 18000, and still more preferably 7000 to 16000.

The weight average molecular weights of the plurality of aromatic vinyl polymer blocks (Ar$^b$) contained in block copolymer B may be equal to or different from each other as long as they fall within the above ranges, but are preferably substantially equal to each other. The weight average molecular weight of the aromatic vinyl polymer block (Ar$^b$) is preferably substantially equal to the weight average molecular weight of the aromatic vinyl polymer block (Ar1$^a$) having a relatively small weight average molecular weight of the block copolymer A.

The conjugated diene polymer block (D$^b$) is a polymer block composed of a conjugated diene monomer unit obtained by polymerizing a conjugated diene monomer as a main repeating unit.

The conjugated diene monomer used for constituting the conjugated diene monomer unit of the conjugated diene polymer block (D$^b$) is not particularly limited as long as it is a conjugated diene compound, and examples thereof include the same as those of the conjugated diene polymer block described above. Further, each conjugated diene polymer block in the block copolymer B may be composed of the same conjugated diene monomer unit or may be composed of different conjugated diene monomer units.

The conjugated diene polymer block (D$^b$), as long as the conjugated diene monomer unit is the main repeating unit, may include another monomer unit, as the monomer constituting the monomer unit other than the conjugated diene monomer unit, examples thereof include the same as those of the conjugated diene polymer block described above, and the content thereof and vinyl bond content may be the same as in the conjugated diene polymer block described above. Further, it is preferable that the conjugated diene polymer block (D$^b$) is also substantially composed of only one type or two or more types of conjugated diene monomer units, and it is particularly preferable that it is composed of only isoprene unit.

The weight average molecular weight of the conjugated diene polymer blocks (D$^b$) is preferably 20000 to 200000, more preferably 22500 to 175000, and still more preferably 25000 to 150000.

When the block copolymer B contains a residue of a coupling agent, the weight average molecular weights of the plurality of conjugated diene polymer blocks (D$^b$) may be equal to or different from each other as long as the weight average molecular weights are within the above ranges, but are preferably substantially equal to each other. When the block copolymer B does not contain a residue of a coupling agent, that is, when two diblock structures (Ar$^b$-D$^b$) are bonded to each other via a single bond, the sum of the weight average molecular weights of the two conjugated diene polymer blocks (D$^b$) is preferably substantially equal to the weight average molecular weight of the conjugated diene polymer block (D$^a$) of the block copolymer A.

Note that, when an aromatic vinyl-conjugated diene-aromatic vinyl block copolymer produced without using a coupling agent is used as the block copolymer B, the conjugated diene polymer block contained therein becomes one in which all monomer units are directly bonded, and it cannot be said that the block is composed of two conjugated diene polymer blocks (D$^b$) on a substantial basis. However, in the present invention, it is assumed that even such a conjugated diene polymer block is conceptually one in which two conjugated diene polymer blocks (D$^b$) having substantially equal weight-average molecular weights are bonded together by a single bond. Therefore, for example, in the block copolymer B which is an aromatic vinyl-conjugated diene-aromatic vinyl block copolymer produced without using a coupling agent, when the conjugated diene polymer block has a weight average molecular weight of 100000 as a whole, it is assumed that the weight average molecular weights of two conjugated diene polymer blocks are 50000.

The weight average molecular weight of the block copolymer B (that is, the weight average molecular weight of the entire block copolymer B) is preferably 65000 to 800000, more preferably 70000 to 700000.

The content of the aromatic vinyl monomer unit of the block copolymer B is preferably 10 to 35% by weight, more preferably 12 to 32% by weight, and still more preferably 15 to 30% by weight, with respect to the total monomer unit constituting the block copolymer B.

The block copolymer B may be obtained by a production method in which a polymer block is formed by sequentially polymerizing an aromatic vinyl monomer, a conjugated diene monomer, and then an aromatic vinyl monomer in this order, or may be obtained by a production method in which an aromatic vinyl monomer and a conjugated diene monomer are polymerized to form a diblock chain, and a diblock chain and a coupling agent are reacted with each other to perform coupling. Therefore, the block copolymer B may be one containing no residue of a coupling agent or one containing a residue of a coupling agent.

However, since more excellent creep resistance and adhesion can be obtained, it is preferable that the block copolymer B is obtained by a production method in which a polymer block is formed by sequentially polymerizing an aromatic vinyl monomer, a conjugated diene monomer, and then an aromatic vinyl monomer in this order. Therefore, it is preferable that the block copolymer B does not contain a residue of a coupling agent. Details of such a production method will be described later.

(Block copolymer C)

The block copolymer according to the present invention may further contain a block copolymer C as a block copolymer. The block copolymer C is a copolymer represented by the following general formula (C):

General formula (C):     $Ar^c$-$D^c$

(In the formula, $Ar^c$ represents an aromatic vinyl polymer block and $D^c$ represents a conjugated diene polymer block.

The aromatic vinyl polymer block ($Ar^c$) is a polymer block composed of an aromatic vinyl monomer unit obtained by polymerizing an aromatic vinyl monomer as a main repeating unit.

The aromatic vinyl monomer used for constituting the aromatic vinyl monomer unit of the aromatic vinyl polymer block ($Ar^c$) is not particularly limited as long as it is an aromatic vinyl compound, and examples thereof include the same as those of the aromatic vinyl polymer block described above.

The aromatic vinyl polymer block ($Ar^c$), as long as the aromatic vinyl monomer unit is the main repeating unit, may include another monomer unit, as the monomer constituting the monomer unit other than the aromatic vinyl monomer unit, examples thereof include the same as those of the aromatic vinyl polymer block described above, and the content thereof may be the same as in the aromatic vinyl polymer block described above. Further, it is preferable that the aromatic vinyl polymer block ($Ar^c$) is also substantially composed of only one type or two or more types of aromatic vinyl monomer units, and it is particularly preferable that it is composed of only styrene unit.

The weight average molecular weight of the aromatic vinyl polymer blocks ($Ar^c$) is preferably 5000 to 20000, more preferably 6000 to 18000, and still more preferably 7000 to 16000.

The weight average molecular weight of the aromatic vinyl polymer block ($Ar^c$) is preferably substantially equal to the weight average molecular weight of the aromatic vinyl polymer block ($Ar1^a$) of the block copolymer A.

The conjugated diene polymer block ($D^c$) is a polymer block composed of a conjugated diene monomer unit obtained by polymerizing a conjugated diene monomer as a main repeating unit.

The conjugated diene monomer used for constituting the conjugated diene monomer unit of the conjugated diene polymer block ($D^c$) is not particularly limited as long as it is a conjugated diene compound, and examples thereof include the same as those of the conjugated diene polymer block described above.

Further, the conjugated diene polymer block ($D^c$), as long as the conjugated diene monomer unit is the main repeating unit, may include another monomer unit, as the monomer constituting the monomer unit other than the conjugated diene monomer unit, examples thereof include the same as those of the conjugated diene polymer block described above, and the content thereof and vinyl bond content may be the same as in the conjugated diene polymer block described above. Further, it is preferable that the conjugated diene polymer block ($D^c$) is also substantially composed of only one type or two or more types of conjugated diene monomer units, and it is particularly preferable that it is composed of only isoprene unit.

The vinyl bond content of the conjugated diene polymer block ($D^c$) is preferably substantially equal to the vinyl bond content of the conjugated diene polymer block ($D^a$) of the block copolymer A.

The weight average molecular weight of the conjugated diene polymer blocks ($D^c$) is preferably 20000 to 400000, more preferably 25000 to 350000, and still more preferably 30000 to 300000.

The weight average molecular weight of the conjugated diene polymer block ($D^c$) is preferably substantially equal to the weight average molecular weight of the conjugated diene polymer block ($D^a$) of the block copolymer A.

The weight average molecular weight of the block copolymer C (that is, the weight average molecular weight of the entire block copolymer C) is preferably 65000 to 800000, more preferably 70000 to 700000.

The content of the aromatic vinyl monomer unit of the block copolymer C is preferably 10 to 35% by weight, more preferably 12 to 32% by weight, and still more preferably 15 to 30% by weight, with respect to the total monomer unit constituting the block copolymer C.

The hot-melt adhesive according to the present invention preferably contains a block copolymer A as the block copolymer, more preferably contains a block copolymer A and at least one selected from the group consisting of block copolymer B and block copolymer C, and further preferably contains a block copolymer A and a block copolymer B.

The content of the block copolymer A in the block copolymer used in the hot-melt adhesive according to the present invention is preferably 10 to 100% by weight, more preferably 20 to 100% by weight, and still more preferably 30 to 100% by weight, with respect to the total weight of the block copolymer.

In addition, in the block copolymer used in the hot-melt adhesive according to the present invention, the content ratio of the block copolymer A and at least one selected from the group consisting of the block copolymer B and the block copolymer C is preferably 10 to 90/90 to 10, more preferably 20 to 80/80 to 20, and still more preferably 30 to 75/70 to 25 in terms of weight ratio (A/B+C).

The block copolymer can be produced according to a conventional method, and examples of the most general production method include a method in which an aromatic vinyl monomer and a conjugated diene monomer are polymerized by an anion living polymerization method to form a polymer block, and if necessary, a coupling agent is reacted to perform coupling. In addition, a method of forming a polymer block by sequentially polymerizing an aromatic vinyl monomer, a conjugated diene monomer, and then an aromatic vinyl monomer in this order may be used.

In addition, in the present invention, it is also possible to use a commercially available block copolymer, and for example, "Quintac (registered trademark)" (manufactured by Zeon Corporation), "KRATON (registered trademark)" (manufactured by Clayton Corporation), "JSR-SIS (registered trademark)" (manufactured by JSR Corporation), "Vector (registered trademark)" (manufactured by DEXCO polymers), "Asaprene (registered trademark)", "Tufprene (registered trademark)", "Tuftec (registered trademark)" (manufactured by Asahi Kasei Chemicals Corporation), "Septon (registered trademark)" (manufactured by Kuraray Co., Ltd.), and the like may be used.

In addition, a method for producing a block copolymer containing two or more of any of the block copolymers A to C is not particularly limited. For example, it can be produced by separately producing each polymer according to a conventional polymerization method and, if necessary, blending other polymer components and the like, and then mixing them according to a conventional method such as kneading or solution mixing. It is also possible to simultaneously produce the block copolymer A and at least one selected from the group consisting of block copolymer B and block copolymer C by a production method described later.

The block copolymer containing the block copolymer A is preferably produced by a method in which a polymer block is formed by sequentially polymerizing an aromatic vinyl monomer, a conjugated diene monomer, and then an aromatic vinyl monomer in this order.

More particularly, the block copolymer A is preferably produced by the following production method. In other words, preferred is a production method comprising:

a first polymerization step of obtaining a solution containing an aromatic vinyl polymer block chain by polymerizing a monomer containing an aromatic vinyl monomer in a solvent in the presence of a polymerization initiator,

a second polymerization step of obtaining a solution containing a diblock chain by adding a monomer containing a conjugated diene monomer to the solution containing the aromatic vinyl polymer block chain and performing a polymerization,

a third polymerization step of obtaining a solution containing a block copolymer A by adding a monomer containing an aromatic vinyl monomer to the solution containing the diblock chain and performing a polymerization.

In the production method above, first, monomer including an aromatic vinyl monomer as the main component is polymerized in a solvent in the presence of a polymerization initiator (first polymerization step). The polymerization initiator to be used can be organic alkali metal compounds, organic alkaline earth metal compounds, organic lanthanoid rare earth metal compounds, and the like, which are known to usually have anionic polymerization activity to aromatic vinyl monomers and conjugated diene monomers. For the organic alkali metal compounds, particularly suitably used are organic lithium compounds having one or more lithium atoms in the molecule. Specific examples thereof include organic monolithium compounds such as ethyllithium, n-propyllithium, isopropyllithium, n-butyllithium, sec-butyllithium, t-butyllithium, hexyllithium, phenyllithium, stilbenelithium, dialkylaminolithium, diphenylaminolithium, and ditrimethylsilylaminolithium; organic dilithium compounds such as methylenedilithium, tetramethylenedilithium, hexamethylenedilithium, isoprenyldilithium, and 1,4-dilithio-ethylcyclohexane; organic trilithium compounds such as 1,3,5-trilithiobenzene; and the like. Among these, organic monolithium compounds are particularly suitably used.

Examples of the organic alkaline earth metal compounds used as the polymerization initiator include n-butylmagnesium bromide, n-hexylmagnesium bromide, ethoxycalcium, calcium stearate, t-butoxystrontium, ethoxybarium, isopropoxybarium, ethylmercaptobarium, t-butoxybarium, phenoxybarium, diethylaminobarium, barium stearate, ethylbarium, and the like. Specific examples of other polymerization initiators include those which form a homogeneous system in an organic solvent to have living polymerizability, such as composite catalysts of lanthanoid rare earth metal compounds (including neodymium, samarium, gadolinium, and the like)/alkylaluminum/alkylaluminum halide/alkylaluminum hydride; metallocene catalysts containing titanium, vanadium, samarium, gadolinium, or the like. These polymerization initiators may be used alone or in combination as a mixture.

The amount of the polymerization initiator to be used may be determined according to the target molecular weight and is not particularly limited. The amount is preferably 0.01 to 20 mmol, more preferably 0.05 to 15 mmol, still more preferably 0.1 to 10 mmol with respect to 100 g of the total monomers.

The solvent used in polymerization can be any solvent inactive to the polymerization initiator. For example, a linear hydrocarbon solvent, a cyclic hydrocarbon solvent, or a mixed solvent thereof is used. Examples of the linear hydrocarbon solvent include linear alkanes and alkenes having 4 to 6 carbon atoms such as n-butane, isobutane, 1-butene, isobutylene, trans-2-butene, cis-2-butene, 1-pentene, trans-2-pentene, cis-2-pentene, n-pentane, isopentane, neopentane, and n-hexane. Specific examples of the cyclic hydrocarbon solvent include aromatic compounds such as benzene, toluene, and xylene; alicyclic hydrocarbon compounds such as cyclopentane and cyclohexane. These solvents may be used alone or in combination as a mixture.

Although not particularly limited, the amount of the solvent used in polymerization is preferably set such that the content of the block copolymer in the finally obtained block copolymer solution falls within the range of preferably 5 to 60% by weight, more preferably 10 to 55% by weight, particularly 20 to 50% by weight.

A Lewis base compound may be added to a reactor used in polymerization to control the structures of the polymer blocks. Examples of the Lewis base compound include ethers such as tetrahydrofuran, diethyl ether, dioxane, ethylene glycol dimethyl ether, ethylene glycol dibutyl ether, diethylene glycol dimethyl ether, and diethylene glycol dibutyl ether; tertiary amines such as tetramethylethylenediamine, trimethylamine, triethylamine, pyridine, and quinuclidine; alkali metal alkoxides such as potassium t-amyloxide and potassium t-butyloxide; phosphines such as triphenylphosphine; and the like. These Lewis base compounds may be used alone or in combination, and are appropriately selected in the range not impairing the object of the present invention.

The Lewis base compound can be added to the polymerization reaction at any timing, which may be appropriately determined according to the structures of the block copolymers. For example, the Lewis base compounds may be preliminarily added before polymerization is started, or may be added after the polymer blocks are partially polymerized. Furthermore, the Lewis base compound may be preliminarily added before polymerization is started, and may be further added after the polymer blocks are partially polymerized.

The temperature for the polymerization reaction is preferably 10 to 150°C, more preferably 30 to 130°C, still more preferably 40 to 90°C. Although the time needed for polymerization varies according to the condition, the time is usually within 48 hours, preferably 0.5 to 10 hours. The polymerization pressure is not particularly limited, and polymerization may be performed at a pressure enough to maintain the monomer and the solvent in liquid phases at a polymerization temperature in the above range.

A solution containing aromatic vinyl polymer block chains can be prepared by polymerizing monomer including an aromatic vinyl monomer as the main component in the presence of the polymerization initiator under the condition described above. The aromatic vinyl polymer block chain produced through polymerization usually has active terminal. The resulting aromatic vinyl polymer block chain will form the aromatic vinyl polymer block (Ar1$^a$). For this reason, the amount of the monomer used in the first polymerization step may be determined according to the weight average molecular weight of the aromatic vinyl polymer block (Ar1$^a$).

In the next step, monomer including a conjugated diene monomer as the main component is added to the solution containing aromatic vinyl polymer block chain prepared in the first polymerization step to perform polymerization (second polymerization step). Thereby, a solution containing diblock chain can be prepared. The diblock chain produced through polymerization usually has active terminal. In the diblock chain produced through the second polymerization step, the polymer chain produced through the first polymerization step which will form the aromatic vinyl polymer block (Ar1$^a$) is bonded to the polymer chain which will form the conjugated diene polymer block (D$^a$). For this reason, the amount of the monomer used in the second polymerization step may be determined according to the weight average molecular weight of the conjugated diene polymer block (D$^a$). The temperature for the polymerization reaction, the polymerization time, and the polymerization pressure may be controlled within the same ranges as those in the first polymerization step.

In the next step, monomer including an aromatic vinyl monomer as the main component is added to the solution containing diblock chain prepared in the second polymerization step to perform polymerization (third polymerization step). Thereby, a solution containing a block copolymer A represented by general formula (A) : Ar1$^a$-D$^a$-Ar2$^a$ can be prepared. In the block copolymer A produced through the third polymerization step, the polymer chain produced in the second polymerization step which will form the aromatic vinyl polymer block (Ar1$^a$) and the conjugated diene polymer block (D$^a$) is further bonded to the polymer chain which will form the aromatic vinyl polymer block (Ar2$^a$). For this reason, the amount of the monomer used in the third polymerization step may be determined according to the weight average molecular weight of the aromatic vinyl polymer block (Ar2$^a$). The temperature for the polymerization reaction, the polymerization time, and the polymerization pressure may be controlled within the same ranges as those in the first polymerization step.

After the third polymerization step, a polymer component may be recovered from the solution containing the block copolymer A (recovery step). The method of recovery may be according to a conventional method, and is not particularly limited. For example, after completion of the reaction, if necessary, a polymerization terminator such as water, methanol, ethanol, propanol, hydrochloric acid, or citric acid is added, and if necessary, an additive such as an antioxidant is further added, and then a known method such as a drying method or a steam stripping is applied directly to the solution to be recovered. When the polymer component is recovered as a slurry by applying steam stripping or the like, the polymer component may be dehydrated using an optional dehydrator such as an extruder type squeezer to obtain a crumb having a moisture content of a predetermined value or less, and the crumb may be dried using an optional dryer such as a band dryer and an expansion extruder. The block copolymer obtained as described above may be processed into a pellet shape or the like according to a conventional method and then used.

As described above, the block copolymer containing the block copolymer A can be produced.

Further, by carrying out the third polymerization step as follows, a block copolymer further containing the block copolymer B can be produced in addition to the block copolymer A. That is, the third polymerization step is carried out by a production method comprising:

a step 1 of a third polymerization step of obtaining a solution containing a triblock chain by adding a monomer containing an aromatic vinyl monomer to the solution containing the diblock chain obtained in the second polymerization step and performing a polymerization,

a polymerization termination step by adding, to the solution containing the triblock chain, a polymerization terminator in an amount which becomes less than 1 molar equivalents with respect to an active terminal in the triblock chain, and a part of the active terminal is deactivated to obtain a solution containing the block copolymer B and the triblock chain,

a step 2 of a third polymerization step of obtaining a solution containing the block copolymer A and the block copolymer B by adding a monomer containing an aromatic vinyl monomer to the solution containing the triblock chain and the block copolymer B and performing a polymerization.

In the step 1 of the third polymerization step, a monomer containing an aromatic vinyl monomer as a main component is added to the solution containing the diblock chain obtained in the second polymerization step, and polymerization is performed. Thus, a solution containing a triblock chain can be obtained. Note that the triblock chain obtained by polymerization is usually one having an active terminal. Further, in the triblock chain produced in the step 1 of the third polymerization step, the polymer chain produced in the second polymerization step which will form the aromatic vinyl polymer block (Ar1$^a$) or the aromatic vinyl polymer block (Ar1$^b$) and the conjugated diene polymer block (D$^a$) or the conjugated diene polymer block (D$^b$) is further bonded to the polymer chain which will form a part of the aromatic vinyl polymer block (Ar2$^a$) or the aromatic vinyl polymer block (Ar2$^b$). For this reason, the amount of the monomer used in the step 1 of the third polymerization step may be determined according to the weight average molecular weight of the aromatic vinyl polymer block (Ar2$^b$). The temperature for the polymerization reaction, the polymerization time, and the polymerization pressure may be controlled within the same ranges as those in the first polymerization step.

Then, a polymerization terminator is added to the solution containing the triblock chain obtained in the step 1 of the third polymerization step in an amount which becomes less than 1 molar equivalents with respect to the active terminal in the triblock chain (termination step). Thus, a part of the active terminal in the triblock chain is deactivated to obtain a block copolymer in which the active terminal is deactivated. This block copolymer is a block copolymer B represented by the general formula (B) : (Ar$^b$-D$^b$)$_n$-X, wherein X is a single bond and "n" is 2.

The polymerization terminator is not particularly limited as long as it can react with the active terminal to deactivate the active terminal and does not react with another active terminal after reacting with the one active terminal, but is preferably a polymerization terminator which is a compound which does not contain a halogen atom from the viewpoint of suppressing moisture absorption of the obtained copolymer, and among them, a polymerization terminator which generates a metal alkoxide, a metal aryl oxide, or a metal hydroxide when reacted with the active terminal is particularly preferred. Examples of the compound particularly preferably used as the polymerization terminator include water, a mono-valent alcohol such as methanol and ethanol, and a mono-valent phenols such as phenol and cresol.

The amount of the polymerization terminator to be used is determined according to the weight ratio of the block copolymer A and the block copolymer B, and is not particularly limited as long as it is an amount which becomes less than 1 molar equivalents with respect to the active terminal in the triblock chain, but is usually preferably in a range in which the polymerization terminator becomes 0.18 to 0.91 molar equivalents with respect to the active terminal and is in a range of 0.35 to 0.80 molar equivalents.

As described above, when a polymerization terminator is added to the solution containing the triblock chain having the active terminal in an amount of less than 1 molar equivalents with respect to the active terminal thereof, a part of the triblock chains having the active terminals is deactivated and a polymer in which its active terminal has been deactivated becomes a block copolymer B. Then, the remaining portion of the triblock chains having an active terminal which has not reacted with the polymerization terminator remains unreacted in the solution.

Then, a monomer containing an aromatic vinyl monomer as a main component is added to the solution containing the triblock chain and the block copolymer B obtained in the termination step and polymerization is performed (step 2 of the third polymerization step). As a result, it is possible to obtain a solution containing the block copolymer A represented by the general formula (A): $Ar1^a$-$D^a$-$Ar2^a$ and the block copolymer B. The block copolymer A produced in the step 2 of the third polymerization step is one in which a polymer chain which forms a part of an aromatic vinyl polymer block ($Ar2^a$) is further bonded to the active terminal of the triblock chain obtained in the step 1 of the third polymerization step. For this reason, the amount of the monomer used in the step 2 of the third polymerization step may be determined according to the weight average molecular weight of the aromatic vinyl polymer block ($Ar2^a$). The temperature for the polymerization reaction, the polymerization time, and the polymerization pressure may be controlled within the same ranges as those in the first polymerization step.

After the step 2 of the third polymerization step, the polymer component may be recovered from the solution containing the block copolymer A and the block copolymer B (recovery step). The method of recovery is as described above.

As described above, a block copolymer containing the block copolymer A and the block copolymer B can be produced.

Further, the above production method may further comprise a termination step of adding a polymerization terminator to the solution containing the diblock chain obtained in the second polymerization step in an amount of less than 1 molar equivalents with respect to an active terminal in the diblock chain, and deactivating a part of the active terminal to obtain a solution containing the block copolymer C and the diblock chain. Thus, a part of the active terminal in the diblock chain is deactivated to obtain a block copolymer in which the active terminal is deactivated. This block copolymer becomes the block copolymer C represented by the general formula (C): $Ar^c$-$D^c$, and finally, a block copolymer further including the block copolymer C in addition to the block copolymer A can be produced.

As the polymerization terminator, those described above can be used. The amount of the polymerization terminator to be used is determined according to the weight ratio of the block copolymer A and the block copolymer C, and is not particularly limited as long as it is an amount which becomes less than 1 molar equivalents with respect to the active terminal in the diblock chain, but is usually preferably in a range in which the polymerization terminator becomes 0.18 to 0.91 molar equivalents with respect to the active terminal and in a range of 0.35 to 0.80 molar equivalents.

As described above, when a polymerization terminator is added to the solution containing the diblock chain having the active terminal in an amount of less than 1 molar equivalents with respect to the active terminal thereof, a part of the diblock chains having the active terminals is deactivated and a polymer in which its active terminal has been deactivated becomes a block copolymer C. Then, the remaining portion of the diblock chains having an active terminal which has not reacted with the polymerization terminator remains unreacted in the solution.

In addition, the above production method may further comprise a coupling step of adding a coupling agent to the solution containing the diblock chain produced in the second polymerization step in an amount of less than 1 molar equivalents with respect to the active terminal of the diblock chain to obtain a solution containing the block copolymer B and the diblock chain. Thereby, the active end of the diblock chain and the coupling agent react with each other, and two or more diblock chains are coupled via the residues of the coupling agent to form a block copolymer B represented by the general formula (B) : $(Ar^b$-$D^b)_n$-X, wherein X is the residue of the coupling agent and "n" is an integer of 2 or more. Then, finally, a block copolymer further containing the block copolymer B in addition to the block copolymer A can be produced.

The coupling agent is not particularly limited as long as it has two or more functional groups in one molecule capable of reacting with the active terminal in the diblock chain, but a coupling agent having 2 to 4 functional groups in one molecule capable of reacting with the active terminal in the diblock chain is preferred. Further, the coupling agent preferably contains a silicon atom, and more preferably a silane halide and an alkoxysilane.

As a coupling agent having two functional groups in one molecule capable of reacting with the active terminal of the diblock chain (two functional coupling agent), for example, two functional halogenated silanes such as dichlorosilane, monomethyldichlorosilane, and dimethyldichlorosilane; two functional alkoxysilanes such as diphenyldimethoxysilane, and diphenyldiethoxysilane; two functional halogenated alkanes such as dibromoethane, methylene chloride, and dibromomethane; two functional tin halides such as dichlorotin, monomethyldichlorotin, dimethyldichlorotin, monoethyldichlorotin, monobutyldichlorotin, and dibutyldichlorotin; dibromobenzene, benzoic acid, CO, 2-chloropropene, and the like may be used. Among these, two functional silane halides or two functional alkoxysilanes is particularly preferably used. As the two functional coupling agents, one kind may be used alone, or two or more kinds may be used in combination.

As a coupling agent having three functional groups in one molecule capable of reacting with the active terminal of the diblock chain (three functional coupling agent), for example, three functional halogenated alkanes such as trichloroethane, and trichloropropane; three functional halogenated silanes such methyltrichlorosilane, and ethyltrichlorosilane; three functional alkoxysilanes such as methyltrimethoxysilane, phenyltrimethoxysilane, and phenyltriethoxysilane; and the like may be mentioned. As the three functional coupling agents, one kind may be used alone, or two or more kinds may be used in combination.

As a coupling agent having four functional groups in one molecule capable of reacting with the active terminal of the diblock chain (four functional coupling agent), for example, four functional halogenated alkanes such as carbon tetrachloride, carbon tetrabromide, and tetrachloroethane; four functional halogenated silanes such as tetrachlorosilane, and tetrabromosilane; four functional alkoxysilanes such as tetramethoxysilane, and tetraethoxysilane; four functional tin halides such as tetrachlorotin, and tetrabromotin; and the like may be mentioned. As the four functional coupling agents, one kind may be used alone, or two or more kinds may be used in combination.

The number of "n" of the block copolymer B, that is, the number of branches of the block copolymer B, can be controlled by adjusting the type of the coupling agent, the amount thereof used, the timing of the addition thereof, the amount of the Lewis base compound used, and the like. Also, a reaction terminator such as methanol can be used to adjust the coupling ratio to adjust the number of branches of the block copolymer B. Further, by using two or more kinds of coupling agents having different numbers of functional groups capable of reacting with the active terminal in the diblock chain in combination, it is also possible to adjust the number of branches of the block copolymer B. Further, by these means, the diblock chain produced in the second polymerization step may be left unreacted and finally recovered as the block copolymer C.

The amount of the coupling agent to be used is adjusted to an appropriate amount according to the number of branches of the target block copolymer B. The amount of the coupling agent to be used is preferably 0.05 to 0.9 molar equivalents, more preferably 0.20 to 0.70 molar equivalents, with respect to the active terminal in the diblock chain.

The reaction temperature is preferably 10 to 150°C, more preferably 30 to 130°C, and still more preferably from 40 to 90°C. The time required for the reaction varies depending on the conditions, but is usually within 48 hours, preferably 0.5 to 10 hours.

As described above, when a coupling agent is added to the solution containing the diblock chain having the active terminal in an amount of less than 1 molar equivalents with respect to the active terminal thereof, a part of the diblock chain having the active terminal reacts with the coupling agent to become the block copolymer B. Then, the remaining portion of the diblock chain having the active end that has not reacted with the polymerization terminator remains unreacted in the solution.

(Tackifying resin)

The hot-melt adhesive according to the present invention prefrebly further contains a tackifying resin. The tackifying resin is not particularly limited, but conventionally known tackifying resins can be used.

Examples of the tackifying resin include rosin; modified rosins such as hydrogenated rosins, disproportionated rosins, and dimerized rosins; esterified products of polyhydric alcohols such as glycol, glycerol, and pentaerythritol with rosin or modified rosins; terpene resins such as $\alpha$-pinene, $\beta$-pinene, and dipentene (limonene) resins; aliphatic, aromatic, and alicyclic hydrocarbon resins and aliphatic-aromatic copolymerized hydrocarbon resins or hydrides thereof; phenol resins; coumarone-indene resins; and the like. Among these, suitably used are aliphatic hydrocarbon resins or aliphatic-aromatic copolymerized hydrocarbon resins which have high miscibility with the copolymer. These tackifying resins may be used or in combination.

Examples of the hydrocarbon resins include a hydrocarbon resin obtained by polymerizing a hydrocarbon monomer mixture containing an aromatic monoolefin and a hydride thereof.

Specific examples of the aromatic monoolefin contained in the hydrocarbon monomer mixture used for obtaining the hydrocarbon resin include styrene, $\alpha$-methylstyrene, isopropenyltoluene, vinyltoluene, and indene, and among them, styrene is particularly preferably used. In addition, it is preferable that the hydrocarbon monomer mixture contains an aliphatic monoolefin having 4 to 8 carbon atoms and an aliphatic diolefin having 4 to 5 carbon atoms as a component other than an aromatic monoolefin. Specific examples of aliphatic monoolefins having 4 to 8 carbon atoms include chain monoolefins such as butenes, pentenes, methylbutenes, methylpentenes, and diisobutylenes, and cyclic monoolefins such as cyclopentene, cyclohexene, methylcyclopentenes, and the like. Specific examples of the aliphatic diolefin having 4 to 5 carbon atoms include chain diolefins such as 1,3-butadiene, isoprene and 1,3-pentadiene, and cyclic diolefins such as cyclopentadiene.

Such hydrocarbon monomer mixtures can generally be obtained as naphtha decomposition fractions of -20 to 60°C, but those obtained by adding an aliphatic monoolefin fraction and/or an aromatic monoolefin fraction to this fraction may be used as a hydrocarbon monomer mixture. Although there is no particular limitation on the method of polymerizing the hydrocarbon monomer mixture, it is usually polymerized using an acidic metal halide catalyst such as aluminum chloride.

The aromatic monomer unit content of the tackifying resin is preferably 1 to 70% by weight, more preferably 2 to 60% by weight, and still more preferably from 5 to 50% by weight.

In addition, in the hot-melt adhesive according to the present invention, it is also possible to use a so-called non-aromatic tackifying resin having an aromatic monomer unit content of less than 1% by weight as a tackifying resin in combination. Specific examples of the non-aromatic tackifying resin include a rosin-based tackifying resin, a terpene-based tackifying resin, an aliphatic tackifying resin, an alicyclic tackifying resin, and a hydride thereof. In addition, when a non-aromatic tackifying resin is used in combination, the aromatic monomer unit content as the entire tackifying resin may be set within the range described above.

The weight average molecular weight of the tackifying resin is not particularly limited, but is usually in the range of 300 to 6000, and preferably in the range of 500 to 5000. Further, the softening point of the tackifying resin is not particularly limited, but is usually in the range of 50 to 160°C, preferably in the range of 60 to 120°C.

The content of the tackifying resin in the hot-melt adhesive according to the present invention is not particularly limited, but is usually 30 to 400 parts by weight, preferably 50 to 350 parts by weight, and more preferably 70 to 300 parts by weight with respect to 100 parts by weight of the block copolymer.

The hot-melt adhesive according to the present invention may further contain a softening agent. Conventionally known softening agents can be used as the softening agent. Specifically, an aromatic, paraffinic or naphthenic extended oil (extender oil); a liquid polymer such as polybutene, polyisobutylene, or the like may be used. The amount of the softening agent to be used is not particularly limited, but is 500 parts by weight or less, preferably 10 to 350 parts by weight, and more preferably 30 to 250 parts by weight with respect to 100 parts by weight of the block copolymer. Note that, as the softening agents, one kind may be used alone, or two or more kinds may be used in combination.

The hot-melt adhesive according to the present invention may contain an antioxidant. As the antioxidants, for example, hindered phenol-based compounds such as pentaerythritol tetrakis[3-(3,5-di-t-butyl-4-hydroxyphenyl)propionate], 2,6-di-t-butyl-p-cresol, di-t-butyl-4-methylphenol; thiodicarboxylate esters such as dilaurylthiopropionate; phosphites such as tris(nonylphenyl)phosphite; may be used. The content of the antioxidant is not particularly limited, but is usually 10 parts by weight or less, preferably 0.5 to 5 parts by weight, with respect to 100 parts by weight of the block copolymer. Note that, as the antioxidants, one kind may be used alone, or two or more kinds may be used in combination.

Further, the hot-melt adhesive according to the present invention may further contain other compounding agents such as a heat stabilizer, an ultraviolet absorber, a filler, and the like. Note that, the hot-melt adhesive according to the present invention is preferably a solvent-free composition containing no solvent.

In obtaining the hot-melt adhesive according to the present invention, a method of mixing the block copolymer and the tackifying resin and various additives is not particularly limited, and examples thereof include a method in which each component is dissolved in a solvent and heat mixed, and then the solvent is removed by heating or the like, and a method in which each component is heat melted and mixed by a kneader or the like.

In using the hot-melt adhesive according to the present invention, the coating method to various members is not particularly limited, but the coating can be performed by, for example, a method such as T die coating, roll coating, multi-bead coating, spray coating, foam coating, or the like.

The hot-melt adhesive according to the present invention is easy to coat at a relatively low temperature and is excellent in creep resistance and adhesion. Therefore, the hot-melt adhesive according to the present invention can be conveniently applied to adhesion of various members, and further, it is possible to perform adhesion with energy saving, good productivity, and high holding power.

The hot-melt adhesive according to the present invention can be applied to various applications, and although its application is not limited, it is suitably used for hot-melt bonding of members which are likely to burn or deterioration at high temperature because of good coatability at relatively low temperature, and in particular, it can be suitably used, in the manufacture of disposable diapers (paper diapers) and sanitary napkins, for bonding of thermoplastic resin sheets and nonwoven fabrics which are constituent members thereof. Further, since the hot-melt adhesive according to the present invention is excellent in creep resistance and adhesion, and is excellent in coatability at a relatively low temperature, it is suitably used as an adhesive agent for various adhesive tapes and labels, and it is possible to provide an adhesive tape and labels which are energy-saving, have high productivity, and have excellent holding power and tackiness.

EXAMPLES

Hereinafter, the present invention will be described specifically by way of Examples and Comparative Examples. In each example, the term "parts" is based on weight unless otherwise specified. Note that the tests and the evaluations were carried out as follows.

[Weight average molecular weight of each block copolymer]

The weight average molecular weight was determined as a molecular weight in terms of polystyrene by high performance liquid chromatography using tetrahydrofuran having a flow rate of 0.35 ml/min as a carrier. The device used was a HLC8320 manufactured by Tosoh, a column connected with three Shodex KF-404HQ manufactured by Showa Denko K. K. (column temperature: 40°C), a differential refractometer and an ultraviolet detector as detectors, and the molecular weight was calibrated at 12 points of standard polystyrenes (500 to 3 million) manufactured by Tosoh.

[Weight ratio of each block copolymer in block copolymer composition]

It was determined from the area ratio of the peak corresponding to each block copolymer of the chart obtained by high performance liquid chromatography described above.

[Weight average molecular weight of styrene polymer block of each block copolymer]

According to the process described in Rubber Chem. Technol., 45, 1295 (1972), the block copolymer was reacted with ozone and reduced with lithium aluminum hydride to decompose the isoprene polymer block of the block copolymer. Specifically, the following procedure was performed. That is, 300 mg of the sample was dissolved in a reaction vessel containing 100 ml of dichloromethane treated with molecular sieves. This reaction vessel was placed and cooled to -25°C in a cooling tank, and ozone generated by an ozone generator was introduced while oxygen was flowed into the reaction vessel at a flow rate of 170 ml/min. After 30 minutes from the start of the reaction, it was confirmed that the reaction was completed by introducing the gas flowing out of the reaction vessel into the aqueous potassium iodide solution. Then, 50 ml of diethyl ether and 470 mg of lithium aluminum hydride were charged into another reaction vessel subjected to nitrogen substitution, and a solution reacted with ozone was slowly added dropwise to this reaction vessel while cooling the reaction vessel with ice water. Then, the reaction vessel was placed in a water bath, and the temperature was gradually increased to reflux at 40°C for 30 minutes. Thereafter, while stirring the solution, dilute hydrochloric acid was added dropwise in small portions to the reaction vessel, and dropping was continued until the generation of hydrogen was hardly recognized. After this reaction, the solid product formed in the solution was filtered off and the solid product was extracted with 100 ml of diethyl ether for 10 minutes. The extract was combined with the filtrate after filtration, and the solvent was distilled off to obtain a solid sample. With respect to the sample thus obtained, the weight average molecular weight was measured according to the method of measuring the weight average molecular weight described above, and the value thereof was defined as the weight average molecular weight of the styrene polymer block.

[Weight average molecular weight of isoprene polymer block of each block copolymer]

From the weight average molecular weight of the block copolymer determined as described above, the weight average molecular weight of the corresponding styrene polymer block was subtracted, and the weight average molecular weight of the isoprene polymer block was determined based on the calculated value.

[Vinyl bond content of isoprene polymer block of each block copolymer]

It was determined based on the measurement of proton NMR.

[Styrene unit content of each block copolymer]

It was determined based on the detection intensity ratio between the differential refractometer and the ultraviolet detector in the measurement of the high performance liquid chromatography. Note that, in advance, copolymers having different styrene unit contents were prepared, and using them, a calibration curve was prepared.

[Melt flow rate of block copolymer]

It was measured in accordance with ISO 1133 (G condition, 200°C, 5 kg).

[Shore A hardness of block copolymer]

It was determined based according to ISO 7619. Details are as follows.
15 g of the block copolymer composition was placed in a press molding machine set at 170°C, preliminarily melted under 0.1 to 0.5 MPa pressure for 1 minute, and then subjected to 20 MPa pressure to form the block copolymer composition into sheets. This procedure was repeated 2 to 3 times to produce a uniform sheet.
The resulting sheet was folded to a size of 4 cm × 4 cm, put into a press molding machine again using a mold of thickness 2 mm, and preliminarily melted under 0.1 to 0.5 MPa pressure for 1 minute, then subjected to 10 MPa pressure to form into a plate shape of thickness 2 mm.
The obtained plate shaped sample was cut to a size of 3 cm × 4 cm, and three of which were stacked to prepare a sample for hardness measurement.
A durometer (type A) was used for the measurement. The hardness measurement sample was placed in predetermined place, the measurement needle was slowly lowered onto the sample, and the indicated vale was read 10 seconds after the needle was lowered. The measurement position was changed and the measurement was performed five times, and the average value thereof was calculated.

[Creep resistance]

(Preparation of coated samples)

A hot-melt adhesive was applied to a yarn rubber by a V-slit coating, and a yarn rubber was stretched and bonded to a nonwoven fabric to be used as a coated sample. The coating temperature was 140°C. The amount applied was 30 gsm. The draw ratio of the yarn rubber was 1.5 times.

(Creep resistance evaluation)

As the sample, one obtained by sticking yarn rubber to the nonwoven fabric was used, and this sample was cut to a length of 250 mm to 300 mm and adhered to a cardboard plate in a fully stretched state. Then, marked with an oily pen at an arbitrary two point such that the rubber length of the test piece stuck was 200 mm, and the rubber was cut at this mark and left at 37.7°C for 4 hours.

After 4 hours, the rubber length was measured and the creep resistance was calculated. The equation for calculating the creep resistance is shown below.

Creep resistance (%) = (200 - length of yarn rubber after 4 hours)/200 × 100

The smaller the creep resistance value, the less the yarn rubber deviates from the nonwoven fabric during the test, indicating that the elasticity holding force is excellent.

[Adhesion]

The adhesion was evaluated by measuring the peel bond strength (N/m) at ordinary temperature at 23°C according to PSTC-1 (180° peel bond test by the U.S. Adhesive Tape Commission) using a rigid polyethylene plate as an adherend. The larger the value, the better the adhesion.

[Production Example 1]

To the pressure-resistant reactor was added 23.3 kg of cyclohexane, 2.2 mmol of N,N,N',N'-tetramethylethylenediamine (hereinafter referred to as "TMEDA"), and 1.20 kg of styrene. While stirring the entire volume at 40°C, 148.1 mmol of n-butyllithium was added. After completion of the addition, the polymerization reaction was carried out for 1 hour by raising the temperature to 50°C. The polymerization conversion of styrene at this time was 100% by weight.

Subsequently, 6.50 kg of isoprene was continuously added to the reactor over a period of 1 hour while the temperature was controlled to keep the temperature between 50 and 60°C. After completion of the addition of isoprene, a polymerization reaction was further carried out for 1 hour. The polymerization conversion of isoprene at this time was 100%.

Then, 1.20 kg of styrene was continuously added over 1 hour, with temperature control to keep 50 to 60°C. After completion of the addition of styrene, a polymerization reaction was further performed for 1 hour to obtain a liquid containing a triblock chain having an active terminal. The polymerization conversion of styrene at this time was 100%.

Then, 110.3 mmol of methanol was added as a polymerization terminator to deactivate a part of the active terminals of the triblock chains and mixed to form styrene-isoprene-styrene triblock copolymer B.

Thereafter, 1.10 kg of styrene was continuously added for 1 hour while temperature control was further carried out to keep the temperature between 50 and 60°C. After completion of the addition of styrene, a polymerization reaction was further performed for 1 hour to obtain a solution containing a triblock chain having an active end. The polymerization conversion of styrene at this time was 100%.

Finally, 296.2 mmol of methanol was added as a polymerization terminator and mixed to deactivate all of the active terminals of the triblock chains to form styrene-isoprene-styrene triblock copolymer A.

The amounts of each reagent used in the reaction are summarized in Table 1. Further, a part of the reaction solution containing the block copolymer composition (1) obtained as described above was taken out, and the block copolymer composition (1) was evaluated according to the above measurement method. The results are shown in Table 2.

To 100 parts (containing 30 parts of the polymer component) of the reaction solution obtained as described above, 0.3 parts of 2,6-di-tert-butyl-p-cresol was added as an antioxidant and mixed, and the mixed solution was added dropwise into hot water heated to 85 to 95°C in small portions to volatilize the solvent to obtain a precipitate, and the precipitate was pulverized and dried by hot air at 85°C, thereby recovering the block copolymer composition (1).

[Production Example 2]

A reaction solution and a block copolymer composition (2) were obtained in the same manner as in Production Example 1, except that the amount of each reagent used for polymerization was changed as shown in Table 1, respectively. The results are shown in Table 2.

[Production Example 3]

To the pressure-resistant reactor, 23.3 kg of cyclohexane, 1.9 mmol of TMEDA and 1.52 kg of styrene were added, and 127.4 mmol of n-butyllithium was added where the mixture was stirred at 40°C, and the mixture was polymerized for 1 hour while raising the temperature to 50°C. The polymerization conversion of styrene was 100%.
Subsequently, 6.50 kg of isoprene was continuously added to the reactor over a period of 1 hour while the temperature was controlled to keep the temperature between 50 and 60°C.
After completion of the addition of isoprene, the mixture was further polymerized for 1 hour, and thereafter, 51 mmol of methanol was added as a polymerization terminator to the reactor to perform a polymerization termination reaction for 1 hours, and a part of the active terminals of the diblock chains having the active terminals was deactivated to form a styrene-isoprene diblock copolymer C.
Thereafter, 1.98 kg of styrene was continuously added over a period of 1 hour while temperature control was performed so as to keep the temperature between 50 and 60°C. After completion of the addition of styrene, it was further polymerized for 1 hour to form a triblock chain. The polymerization conversion of styrene was 100%. Thereafter, 254.8 mmol of methanol was added as a polymerization terminator and mixed to deactivate all of the active terminals of the triblock chains to form a styrene-isoprene-styrene block copolymer A.
The amounts of each reagent used in the reaction are summarized in Table 1. Further, a part of the reaction solution containing the block copolymer composition (3) obtained as described above was taken out, and the block copolymer composition (3) was evaluated according to the above measurement method. The results are shown in Table 2.
Further, the block copolymer composition (3) was recovered in the same manner as in Production Example 1.

[Production Example 4]

To the pressure-resistant reactor, 23.3 kg of cyclohexane, 2.6 mmol of N,N,N',N'-tetramethylethylenediamine (hereinafter referred to as TMEDA) and 1.83 kg of styrene were added, and 175.7 mmol of n-butyllithium was added where the mixture was stirred at 40°C, and the mixture was polymerized for 1 hour while raising the temperature to 50°C. The polymerization conversion of styrene was 100% by weight.
Subsequently, 5.20 kg of isoprene was continuously added to the reactor over a period of 1 hour while the temperature was controlled to keep the temperature between 50 and 60°C.
After completing the addition of isoprene, it was further polymerized for 1 hour. Polymerization conversion rate of isoprene was 100%. Then, 60.7 mmol of dimethyldichlorosilane was added as a coupling agent to perform a coupling reaction for 2 hours to form a styrene-isoprene-styrene coupling block copolymer B.
Thereafter, 2.97 kg of styrene was continuously added over a period of 1 hour while temperature control was performed so as to keep the temperature between 50 and 60°C. After completing the addition of styrene, it was further polymerized for 1 hour to form triblock chain having an active terminal. The polymerization conversion of styrene was 100%. Thereafter, 351.4 mmol of methanol was added as a polymerization terminator and mixed to deactivate all of the active terminals of the triblock chain to form a styrene-isoprene-styrene block copolymer A.
The amounts of each reagent used in the reaction are summarized in Table 1. Further, a part of the reaction solution containing the block copolymer composition (4) obtained as described above was taken out, and the block copolymer composition (4) was evaluated according to the above measurement method. The results are shown in Table 2.
Further, the block copolymer composition (4) was recovered in the same manner as in Production Example 1.

Table 1

**Table 1**

| | | Production Example | | | |
|---|---|---|---|---|---|
| | | 1 | 2 | 3 | 4 |
| Cyclohexane (kg) | | 23.3 | 23.3 | 23.3 | 23.3 |
| TMEDA (mmol) | | 2.2 | 2.3 | 1.9 | 2.6 |
| **n-butyllithium** (mmol) | | 148.1 | 153.7 | 127.4 | 175.7 |
| Styrene (kg) | [First stage of polymerization] | 1.20 | 1.23 | 1.52 | 1.83 |
| Isoprene (kg) | [Second stage of polymerization] | 6.50 | 6.00 | 6.50 | 5.20 |
| Methanol (mmol) | [After second stage of polymerization] | - | - | 51 | - |
| Styrene (kg) | [Third stage of polymerization] | 1.20 | 1.23 | 1.98 | - |
| Dimethyldichlorosilane (mmol) | [After third stage of polymerization] | - | - | - | 60.7 |
| Methanol (mmol) | [After third stage of polymerization] | 110.3 | 111.0 | - | - |
| Styrene (kg) | [Fourth stage of polymerization] | 1.10 | 1.55 | - | 2.97 |
| Methanol (mmol) | [After fourth stage of polymerization] | 296.2 | 307.5 | 254.8 | 351.4 |

Table 2

Table 2

| | | Production Example | | | |
|---|---|---|---|---|---|
| | | 1 | 2 | 3 | 4 |
| Number of composition | | (1) | (2) | (3) | (4) |
| Block copolymer A | | | | | |
|     Weight average molecular weight | [*10^3] | 119 | 120 | 120 | 121 |
|     Weight average molecular weight of Ar1[a] | [*10^3] | 9 | 9 | 13 | 10 |
|     Weight average molecular weight of Ar2[a] | [*10^3] | 44 | 52 | 28 | 63 |
|     Aromatic vinyl monomer unit content | [wt%] | 51 | 57 | 43 | 69 |
|     Vinyl bond content | [wt%] | 7 | 7 | 7 | 7 |
|     Weight average molecular weight of D[a] | [*10^3] | 66 | 59 | 79 | 48 |
| Block copolymer B | | | | | |
|     Weight average molecular weight | [*10^3] | 84 | 77 | - | 116 |
|     Weight average molecular weight of Ar1[b] | [*10^3] | 9 | 9 | - | 10 |
|     Weight average molecular weight of Ar2[b] | [*10^3] | 9 | 9 | - | 10 |
|     Aromatic vinyl monomer unit content | [wt%] | 27 | 29 | - | 26 |
|     Vinyl bond content | [wt%] | 7 | 7 | - | 7 |
|     Weight average molecular weight of D[b] | [*10^3] | 33 | 30 | - | 48 |

(continued)

| Block copolymer C | | | | | | |
|---|---|---|---|---|---|---|
| Weight average molecular weight | [*10^3] | - | - | 91 | - |
| Weight average molecular weight of Ar$^c$ | [*10^3] | - | - | 13 | - |
| Aromatic vinyl monomer unit content | [wt%] | - | - | 19 | - |
| Vinyl bond content | [wt%] | - | - | 7 | - |
| Weight average molecular weight of D$^c$ | [*10^3] | - | - | 79 | - |
| Entire block copolymer (entire composition) | | | | | | |
| Weight average molecular weight | [*10^3] | 95.7 | 97.5 | 111 | 119 |
| Aromatic vinyl monomer unit content | [wt%] | 35 | 40 | 35 | 48 |
| Content of block copolymer A | [wt%] | 35 | 40 | 70 | 50 |
| Content of block copolymer B | [wt%] | 65 | 60 | 0 | 50 |
| Content of block copolymer C | [wt%] | - | - | 30 | 0 |
| Melt flow rate, G condition | [g/10min] | 70 | 120 | 50 | 12 |
| Shore A hardness | | 55 | 60 | 50 | 62 |

Table 3 also shows the results of the evaluation of commercial block copolymers (5) to (8) used in Comparative Examples 2 to 7 described below.

Block copolymer composition (5) is Trade name "Vector 4411" manufactured by DEXCO polymers.

Block copolymer composition (6) is Trade name "Vector 4211" manufactured by DEXCO polymers.

Block copolymer composition (7) is Trade name "Vector 4111" manufactured by DEXCO polymers.

Block copolymer composition (8) is Trade name "Asaprene T439" manufactured by Asahi Kasei Chemicals Corporation, a linear-type styrenebutadiene block copolymer.

Table 3

Table 3

| | | Commercial block copolymer | | | |
|---|---|---|---|---|---|
| Number of composition | | (5) | (6) | (7) | (8) |
| Aromatic vinyl monomer unit content | [wt%] | 44 | 30 | 19 | 43 |
| Content of block copolymer A | [wt%] | 0 | 0 | 0 | 0 |
| Content of block copolymer B | [wt%] | 100 | 100 | 100 | 40 |
| Content of block copolymer C | [wt%] | 0 | 0 | 0 | 60 |
| Melt flow rate, G condition | [g/10min] | 44 | 12 | 10 | 97 |
| Shore A hardness | | 90 | 68 | 50 | 83 |

[Example 1]

25 parts of the block copolymer composition (1) obtained in Production Example 1 were put into a stirring blade-type kneader, and to this, 50 parts of tackifying resin (trade name "Escorez 5300" manufactured by Exxon Mobil Corporation, softening point 105°C, hydrogenated dicyclopentadiene-based resin), 9 parts of the tackifying resin (trade name "Endex 155" manufactured by Eastman Chemical Company, softening point 155°C, polystyrene resin), 15 parts of the softening agent (trade name "Diana Process Oil NS-90S" manufactured by Idemitsu Kosan Co. Ltd., naphthenic oil), and 1 part of the antioxidant (trade name "Irganox 1010" manufactured by BASF SE) were added and the reaction system was replaced with nitrogen gas. Then the mixture was kneaded at 160 to 180°C for 1 hour to prepare a hot-melt adhesive. A part of the obtained hot-melt adhesive was used to evaluate the creep resistance by the method described above. Further, the hot-melt adhesive was heated to 160°C and was coated on a hard polyethylene plate to form a coating film having a thickness of 30 μm, and an adhesion was evaluated for a sample obtained by this. The results are shown in Table 4.

[Examples 2 to 6]

A hot-melt adhesive was prepared in the same manner as in Example 1, except that the formulation was changed as shown in Table 4, and evaluated in the same manner. The results are shown in Table 4.

[Comparative Examples 1 to 7]

A hot-melt adhesive was prepared in the same manner as in Example 1, except that the formulation was changed as shown in Table 4, and evaluated in the same manner. The results are shown in Table 4.

Table 4

Table 4

| | | Example 1 | Example 2 | Example 3 | Example 4 | Example 5 | Example 6 | Comp. Example 1 | Comp. Example 2 | Comp. Example 3 | Comp. Example 4 | Comp. Example 5 | Comp. Example 6 | Comp. Example 7 |
|---|---|---|---|---|---|---|---|---|---|---|---|---|---|---|
| Block copolymer composition (1) | wt% | 25 | | | 60 | | 41 | | | | | | | |
| Block copolymer composition (2) | wt% | | 25 | 40 | | | | | | | | | | |
| **Block copolymer** composition (3) | wt% | | | | | 25 | | | | | | | | |
| Block copolymer composition (4) | wt% | | | | | | | 25 | | | | | | |
| Block copolymer composition (5) | wt% | | | | | | | | 25 | | | | | |
| Block copolymer composition (6) | wt% | | | | | | | | | 25 | | | 41 | |
| Block copolymer composition (7) | wt% | | | | | | | | | | 25 | | | |
| Block copolymer composition (8) | wt% | | | | | | | | | | | 25 | | 41 |
| Escorez 5300 (manufactured by Exxon Mobil Corporation) | wt% | 50 | 50 | 50 | 30 | 50 | 30 | 50 | 50 | 50 | 50 | 50 | 30 | 30 |

22

EP 3 842 504 B1

(continued)

| | | Example 1 | Example 2 | Example 3 | Example 4 | Example 5 | Example 6 | Comp. Example 1 | Comp. Example 2 | Comp. Example 3 | Comp. Example 4 | Comp. Example 5 | Comp. Example 6 | Comp. Example 7 |
|---|---|---|---|---|---|---|---|---|---|---|---|---|---|---|
| Endex 155 (manufactured by Eastman Chemical Company) | wt% | 9 | 9 | 5 | 1 | 9 | 9 | 9 | 5 | 9 | 9 | 9 | 9 | 9 |
| Naphthenic oil | wt% | 15 | 15 | 4 | 8 | 15 | 19 | 15 | 4 | 15 | 15 | 15 | 19 | 19 |
| Antioxidant | wt% | 1 | 1 | 1 | 1 | 1 | 1 | 1 | 1 | 1 | 1 | 1 | 1 | 1 |
| Creep resistance, residual strain | % | 20 | 14 | 8 | 5 | 25 | 8 | 35 | 10 | 45 | 60 | 80 | 50 | 55 |
| Adhesion, to polyehylene | N/m | 320 | 380 | 300 | 280 | 520 | 350 | 230 | 80 | 200 | 310 | 150 | 120 | 100 |

23

EP 3 842 504 B1

As the results shown in Table 4, a hot-melt adhesive containing a block copolymer having a melt flow rate, a content of an aromatic vinyl monomer unit, and a Shore A hardness appropriately adjusted was excellent in creep resistance and also excellent in adhesion (Examples 1 to 6).

Also, in addition to the block copolymer A represented by the general formula (A): $Ar1^a$-$D^a$-$Ar2^a$, the hot-melt adhesive further containing the block copolymer B represented by the general formula (B): $(Ar^b$-$D^b)_n$-$X$ in addition to the block copolymer A represented by the general formula (A): $Ar1^a$-$D^a$-$Ar2^a$ was superior in the creep resistance (Examples 1 to 4 and 6) .

On the other hand, if at least one of the melt flow rate, the content of the aromatic vinyl monomer unit, and the Shore A hardness was too small or too large, a hot-melt adhesive excellent in creep resistance and also excellent in adhesion could not be obtained (Comparative Examples 1 to 7).

**Claims**

1. A hot-melt adhesive comprising a block copolymer composition, wherein

the block copolymer composition has a melt flow rate measured in accordance with ISO 1133 [G condition, 200°C, 5 kg] of 40 to 200 g/10 min,
the block copolymer composition consists of two or more block copolymers,
each of the block copolymers contain an aromatic vinyl polymer block and a conjugated diene polymer block,
the block copolymer composition comprises, as the block copolymer, a block copolymer A represented by the following general formula (A) and at least one selected from the group consisting of a block copolymer B represented by the following general formula (B) and a block copolymer C represented by the following general formula (C),

General formula (A): $Ar1^a$-$D^a$-$Ar2^a$

in the general formula (A), $Ar1^a$ is an aromatic vinyl polymer block having a weight average molecular weight of 5000 to 20000, $Ar2^a$ is an aromatic vinyl polymer block having a weight average molecular weight of 22000 to 400000, and $D^a$ is a conjugated diene polymer block,

General formula (B): $(Ar^b$-$D^b)_n$-$X$

in the general formula (B), $Ar^b$ is an aromatic vinyl polymer block having a weight average molecular weight of 5000 to 20000, $D^b$ is a conjugated diene polymer block, X is a single bond, n is 2, and the sum of the weight average molecular weights of the two conjugated diene polymer blocks $D^b$ is equal to the weight average molecular weight of the conjugated diene polymer block $D^a$ of the block copolymer A,

General formula (C): $Ar^c$-$D^c$

in the general formula (C), $Ar^c$ represents an aromatic vinyl polymer block and $D^c$ represents a conjugated diene polymer block, and the weight average molecular weight of the conjugated diene polymer block $D^c$ is equal to the weight average molecular weight of the conjugated diene polymer block $D^a$ of the block copolymer A,
each of a weight average molecular weight of a polymer block and a weight average molecular weight of an entire block polymer is determined as a value in terms of polystyrene by measurement of high performance liquid chromatography as set forth in the description,
a content of an aromatic vinyl monomer unit in the block copolymer composition is 30 to 80% by weight with respect to the total monomer unit constituting the block copolymer composition, and
the block copolymer composition has a Shore A hardness determined in accordance with ISO 7619 of 65 or less.

2. The hot-melt adhesive according to claim 1, wherein the conjugated diene polymer block contains an isoprene unit.

3. The hot-melt adhesive according to claim 1 or 2, wherein the block copolymer composition comprises, as the block copolymer, the block copolymer A and the block copolymer B.

4. The hot-melt adhesive according to any of claims 1 to 3, further comprising a tackifying resin.

5. The hot-melt adhesive according to claim 4, comprising a non-aromatic tackifying resin having an aromatic monomer unit content of less than 1% by weight as the tackifying resin.

6. Use of the hot-melt adhesive according to any of claims 1 to 5 in the manufacture of a paper diaper.

**Patentansprüche**

1. Heißschmelzklebstoff, umfassend eine Blockcopolymerzusammensetzung, wobei

die Blockcopolymerzusammensetzung eine gemäß ISO 1133 [G-Bedingung, 200°C, 5 kg] gemessene Schmelzflussrate von 40 bis 200 g/10 min aufweist,
die Blockcopolymerzusammensetzung aus zwei oder mehr Blockcopolymeren besteht,
jedes der Blockcopolymere einen aromatischen Vinylpolymerblock und einen konjugierten Dienpolymerblock enthält,
die Blockcopolymerzusammensetzung umfasst: als Blockcopolymer ein Blockcopolymer A, das durch die folgende allgemeine Formel (A) dargestellt wird, und mindestens eines aus der Gruppe bestehend aus einem Blockcopolymer B, das durch die folgende allgemeine Formel (B) dargestellt wird, und einem Blockcopolymer C, das durch die folgende allgemeine Formel (C) dargestellt wird,

Allgemeine Formel (A):    $Ar1^a\text{-}D^a\text{-}Ar2^a$

in der allgemeinen Formel (A) ist $Ar1^a$ ein aromatischer Vinylpolymerblock mit einem gewichtsmittleren Molekulargewicht von 5000 bis 20000, ist $Ar2^a$ ein aromatischer Vinylpolymerblock mit einem gewichtsmittleren Molekulargewicht von 22000 bis 400000 und ist $D^a$ ein konjugierter Dienpolymerblock,

Allgemeine Formel (B):    $(Ar^b\text{-}D^b)_n\text{-}X$

in der allgemeinen Formel (B) ist $Ar^b$ ein aromatischer Vinylpolymerblock mit einem gewichtsmittleren Molekulargewicht von 5000 bis 20000, ist $D^b$ ein konjugierter Dienpolymerblock, ist X eine Einfachbindung, ist n 2 und ist die Summe der gewichtsmittleren Molekulargewichte der beiden konjugierten Dienpolymerblöcke $D^b$ gleich dem gewichtsmittleren Molekulargewicht des konjugierten Dienpolymerblocks $D^a$ des Blockcopolymers A,

Allgemeine Formel (C):    $Ar^c\text{-}D^c$

in der allgemeinen Formel (C) steht $Ar^c$ für einen aromatischen Vinylpolymerblock und steht $D^c$ für einen konjugierten Dienpolymerblock, und das gewichtsmittlere Molekulargewicht des konjugierten Dienpolymerblocks $D^c$ ist gleich dem gewichtsmittleren Molekulargewicht des konjugierten Dienpolymerblocks $D^a$ des Blockcopolymers A,
das gewichtsmittlere Molekulargewicht eines Polymerblocks und das gewichtsmittlere Molekulargewicht eines gesamten Blockpolymers werden jeweils als Wert in Bezug auf Polystyrol durch Messung mittels Hochleistungsflüssigkeitschromatographie, wie in der Beschreibung dargelegt, bestimmt,
der Gehalt an aromatischen Vinylmonomereinheiten in der Blockcopolymerzusammensetzung beträgt 30 bis 80 Gew.-% bezogen auf die Gesamtmonomereinheiten, aus denen die Blockcopolymerzusammensetzung besteht, und
die Blockcopolymerzusammensetzung weist eine Shore-A-Härte gemäß ISO 7619 von 65 oder weniger auf.

2. Heißschmelzklebstoff nach Anspruch 1, wobei der konjugierte Dienpolymerblock eine Isopreneinheit enthält.

3. Heißschmelzklebstoff nach Anspruch 1 oder 2, wobei die Blockcopolymerzusammensetzung als Blockcopolymer das Blockcopolymer A und das Blockcopolymer B umfasst.

4. Heißschmelzklebstoff nach einem der Ansprüche 1 bis 3, der ferner ein klebrigmachendes Harz umfasst.

5. Heißschmelzklebstoff nach Anspruch 4, der ein nichtaromatisches klebrigmachendes Harz mit einem Gehalt an aromatischen Monomereinheiten von weniger als 1 Gew.-% als klebrigmachendes Harz umfasst.

6. Verwendung des Heißschmelzklebstoffs nach einem der Ansprüche 1 bis 5 bei der Herstellung einer Papierwindel.

**Revendications**

1. Adhésif thermofusible comprenant une composition de copolymère séquencé, dans lequel

   la composition de copolymère séquencé a un indice de fluidité mesuré conformément à la norme ISO 1133 [condition G, 200°C, 5 kg] de 40 à 200 g/10 min,
   la composition de copolymère séquencé est constituée de deux ou plusieurs copolymères séquencés,
   chacun des copolymères séquencés contient un bloc de polymère vinylique aromatique et un bloc de polymère diénique conjugué,
   la composition de copolymère séquencé comprend, en tant que copolymère séquencé, un copolymère séquencé A représenté par la formule générale (A) suivante et au moins un copolymère choisi dans le groupe constitué par un copolymère séquencé B représenté par la formule générale (B) suivante et un copolymère séquencé C représenté par la formule générale (C) suivante,

   Formule générale (A) : $Ar1^a$-$D^a$-$Ar2^a$

   dans la formule générale (A), $Ar1^a$ est un bloc de polymère vinylique aromatique ayant un poids moléculaire moyen en poids de 5 000 à 20 000, $Ar2^a$ est un bloc de polymère vinylique aromatique ayant un poids moléculaire moyen en poids de 22 000 à 400 000, et $D^a$ est un bloc de polymère diénique conjugué,

   Formule générale (B) : $(Ar^b$-$D^b)_n$-X

   dans la formule générale (B), $Ar^b$ est un bloc polymère vinylique aromatique ayant un poids moléculaire moyen en poids de 5 000 à 20 000, $D^b$ est un bloc polymère diénique conjugué, X est une liaison simple, n est égal à 2, et la somme des poids moléculaires moyens en poids des deux blocs polymères diéniques conjugués $D^b$ est égale au poids moléculaire moyen en poids du bloc polymère diénique conjugué $D^a$ du copolymère séquencé A,

   Formule générale (C) : $Ar^c$-$D^c$

   dans la formule générale (C), $Ar^c$ représente un bloc polymère vinylique aromatique et $D^c$ représente un bloc polymère diénique conjugué, et le poids moléculaire moyen en poids du bloc polymère diénique conjugué $D^c$ est égal au poids moléculaire moyen en poids du bloc polymère diénique conjugué $D^a$ du copolymère séquencé A,
   le poids moléculaire moyen en poids d'un bloc polymère et le poids moléculaire moyen en poids d'un polymère séquencé entier sont déterminés en tant que valeur en termes de polystyrène par mesure par chromatographie liquide haute performance, comme indiqué dans la description,
   la teneur en unités monomères vinyliques aromatiques dans la composition de copolymère séquencé est comprise entre 30 et 80% en poids par rapport à la totalité des unités monomères constituant la composition de copolymère séquencé, et
   la composition de copolymère séquencé a une dureté Shore A déterminée conformément à la norme ISO 7619 inférieure ou égale à 65.

2. Adhésif thermofusible selon la revendication 1, dans lequel le bloc de polymère diénique conjugué contient un motif isoprène.

3. Adhésif thermofusible selon la revendication 1 ou 2, dans lequel la composition de copolymère séquencé comprend, en tant que copolymère séquencé, le copolymère séquencé A et le copolymère séquencé B.

4. Adhésif thermofusible selon l'une quelconque des revendications 1 à 3, comprenant en outre une résine tackifiante.

5. Adhésif thermofusible selon la revendication 4, comprenant une résine tackifiante non aromatique ayant une teneur en unités monomères aromatiques inférieure à 1% en poids en tant que résine tackifiante.

6. Utilisation de l'adhésif thermofusible selon l'une quelconque des revendications 1 à 5 dans la fabrication d'une couche-culotte en papier.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010254983 A

- US 2004005834 A1

**Non-patent literature cited in the description**

- *Rubber Chem. Technol.*, 1972, vol. 45, 1295